# EUROPÄISCHE PATENTSCHRIFT

(11) **EP 0 626 386 B1**
(45) Veröffentlichungstag und Bekanntmachung des Hinweises auf die Patenterteilung: **04.12.1996**
(21) Anmeldenummer: 94108133.3
(22) Anmeldetag: 26.05.1994
(51) Int. Cl.: C07F 7/21, C07F 7/08, C07D 207/40, C09K 19/40, C08G 77/388

(54) **Chirale Weinsäureimide enthaltende flüssigkristalline Organosiloxane**
Chiral tartaric acid imide containing liquid-crystalline organosiloxanes
Organosiloxanes liquides cristallins contenant des groupements à base d'imides de l'acide tartrique chiraux

(30) Priorität: 27.05.1993 DE 4317704
(43) Veröffentlichungstag der Anmeldung: 30.11.1994
(73) Patentinhaber: Consortium für elektrochemische Industrie GmbH, D-81379 München (DE)
(72) Erfinder: Zahn, Ingo, Dr., D-80797 München (DE); Kreuzer, Franz-Heinrich, Dr., D-82152 Martinsried (DE); Weitzel, Hans-Peter, Dr., D-84571 Reischach (DE)

(56) Entgegenhaltungen:
- EP-A- 0 446 912
- EP-A- 0 466 183
- DE-A- 3 822 121
- US-A- 3 586 699

## Beschreibung

Die vorliegende Erfindung betrifft chirale Weinsäureimide enthaltende flüssigkristalline Organosiloxane, ein Verfahren zu deren Herstellung, deren Verwendung, zu chirale Weinsäureimide enthaltenden flüssigkristallinen Organosiloxanen kondensierbare Organosilane, chirale Weinsäureimide und Mischungen von chirale Weinsäureimide enthaltenden flüssigkristallinen Organosiloxanen mit anderen flüssigkristallinen Materialien.

Verdrillte Flüssigkristallmaterialien besitzen im allgemeinen smektische, nematische oder cholesterische Phasen. Flüssigkristallgemische enthalten häufig eine oder mehrere optisch aktive Komponenten zur Induktion einer chiralen Struktur. Beispielsweise können cholesterische Flüssigkristalle aus einem nematischen Basismaterial und einem oder mehreren optisch aktiven Dotierstoffen bestehen. Dabei erzeugen diese im Nematen entweder eine rechts- oder linkshändige Verdrillung. Cholesterische Flüssigkristalle reflektieren Licht in einem Wellenlängenbereich, für den die Wellenlänge etwa gleich der Helixganghöhe ist (λ = n · p). Das reflektierte Licht ist vollständig zirkular polarisiert. Die Drehrichtung des reflektierten Lichts hängt vom Drehsinn der cholesterischen Helixstruktur ab. Das jeweils entgegengesetzt zirkular polarisierte Licht wird ungeschwächt transmittiert.

Eine große Zahl optisch aktiver Dotierstoffe, die sich mehr oder weniger gut für bestimmmte Zwecke eignen sind in der Literatur beschrieben. So sind aus der US-A 4,996,330 chirale N-substituierte und zweifach an den Hydroxylgruppen mit mesogenen Resten veresterte Weinsäureimide bekannt. Solche Substanzen lassen sich als Zusatzstoffe in elektrooptischen Anzeigevorrichtungen wie TN- oder STN-Zellen verwenden, um die erforderliche Verdrillung zu erreichen.

Polymere bzw. vernetzte cholesterische Flüssigkristalle ermöglichen weitergehende Anwendungen. Aus ihnen können LC-Pigmente mit neuartigen Effekten oder reflektierende Polarisationsfilter und vieles mehr hergestellt werden. Die Einarbeitung von beispielsweise Methacrylsäureester von Cholesterin bzw. dessen Derivaten als Dotierstoffe in flüssigkristalline Polymere ist von H.Finkelmann, H.Ringsdorf et al. in Makromol.Chem. 179, 829-832(1978)) beschrieben.

In beispielsweise EP-A-358 208 sind cyclische Siloxane mit mesogenen Seitengruppen beschrieben, bei denen ein Teil der Seitengruppen mit Methacrylsäure verestert ist. Deshalb lassen sich diese Siloxane auch vernetzen. Die in diese Polymere eingearbeiteten Dotierstoffe sind hydrosilylierte Allyloxybenzoesäureester von Cholesterin bzw. dessen Derivaten. Diese weisen aber nur ein relativ geringes Verdrillungsvermögen ("helical twisting power" oder kurz HTP) auf und müssen daher in großen Mengen (ca. 50%) zugesetzt werden, um Farbeffekte im sichtbaren Bereich hervorzurufen. Durch die große Menge an Dotierstoffen kann die Vernetzergruppendichte nicht sehr hoch gewählt werden. Die chiralen Dotierstoffe sind darüber hinaus verhältnismäßig teuer.

Für den Bau mancher optischer Filter ist eine hohe spektrale Breite des Reflexionslichtes von entscheidender Bedeutung. Die spektrale Breite des Reflexionslichtes läßt sich durch einen großen Anteil aromatischer Verbindungen im Flüssigkristall erhöhen. Cholesterinhaltige Systeme weisen jedoch einen hohen Gehalt an Cyclohexylringen auf. Somit ist die spektrale Breite des Reflexionslichts für diese Anwendungen oft zu gering.

Für manche Anwendungen optischer Filter wäre es ferner erwünscht zirkular polarisiertes Licht wahlweise beider Drehrichtungen zu reflektieren. Diese Möglichkeit bieten die oben genannten cholesterinhaltigen Systeme in der Regel ebenfalls nicht.

Aufgabe der vorliegenden Erfindung war es, flüssigkristalline Organosiloxane bereitzustellen, die anstelle der Cholesterinderivate geeignetere Chiralika mit hoher HTP enthalten, cholesterische Phasen ausbilden können, wahlweise zirkular polarisiertes Licht beider Drehrichtungen reflektieren und sich bei Bedarf vernetzen lassen.

Die Erfindung betrifft flüssigkristalline Organosiloxane, welche pro Molekül mindestens einen Si-C gebundenen chiralen Weinsäureimidrest der allgemeinen Formel (1) 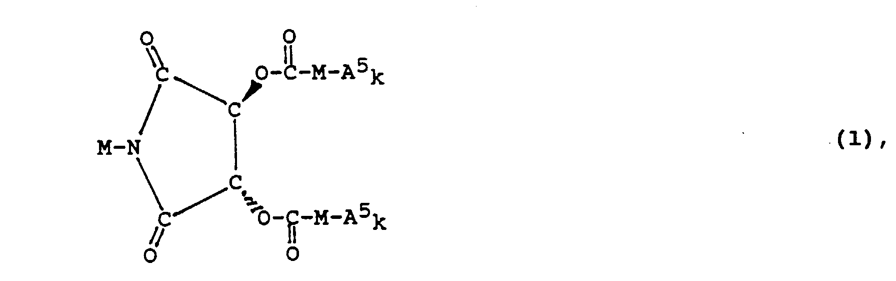 aufweisen, in der
- **M**: einen Rest der allgemeinen Formel 2,

R¹-(X¹ ₐ-A¹ _{b}-A² _{c})_{d}-Zₑ-(-X² _{f}-A³ _{g}-A⁴ ₕ-)ᵢ (2),

bedeutet,
wobei die Organosiloxane aus mindestens zwei Einheiten der allgemeinen Formel 3,

[BₒRₚH_{q}SiO_{(4-o-p-q)/2}] (3),

aufgebaut sind, in der
- **B**: einen Rest der allgemeinen Formel 1, und gegebenenfalls einen Rest der allgemeinen Formel 4,

M-A⁵ ₖ (4),

bedeutet, wobei in den vorstehenden allgemeinen Formeln 1 bis 4
- **R**^{**1**}: einen Rest der Formel CₙHₘ bedeutet, in der
- **n**: eine ganze Zahl im Wert von 0 bis 20,
- **m**: den Wert von 2n besitzt, oder, falls n mindestens 2 bedeutet, auch den Wert von (2n-2) besitzen kann, und in R¹ eine oder mehrere Methyleneinheiten durch Sauerstoffatome ersetzt sein können, welche an Kohlenstoff- und/oder Siliciumatome gebunden sein können,
- **X**^{**1**} und **X**^{**2**}: gleiche oder verschiedene zweibindige Reste aus der Gruppe -O-, -COO-, -CONH-, -CO-, -S-, -C≡C-, -CH=CH-, -CH=N-, -CH₂-, -N=N- und -N=N(O)-,
- **A**^{**1**}**, A**^{**2**}**, A**^{**3**} und **A**^{**4**}: gleiche oder verschiedene zweibindige Reste, nämlich 1,4-Phenylen-, 1,4-Cyclohexylenreste, substituierte Arylene mit 1 bis 10 Kohlenstoffatomen, substituierte Cycloalkylene mit 1 bis 10 Kohlenstoffatomen und Heteroarylene mit 1 bis 10 Kohlenstoffatomen,
- **Z**: gleiche oder verschiedene zwei- bis vierwertige Benzol-, Cyclohexan- oder Cyclopentanreste,
- **A**^{**5**}: gleiche oder verschiedene, gesättigte oder olefinisch ungesättigte Alkyl-, Alkoxy- oder Cycloalkylreste mit jeweils 1 bis 16 Kohlenstoffatomen, Cholestanreste, Cholesterylreste, Halogenatome, Wasserstoffatome, Hydroxyl-, Nitril-, Acryloxy-, (Meth)acryloxy-, (Meth)acryloxyethylenoxy-, (Meth)acryloxydi(ethylenoxy)-, (Meth)acryloxytri(ethylenoxy)-, R- oder S-Tetrahydrofurancarbonsäureester und Trialkylsiloxygruppen, deren Alkylreste jeweils 1 bis 8 Kohlenstoffatome besitzen,
- **R**: gleiche oder verschiedene, gegebenenfalls substituierte C₁- bis C₁₈-Kohlenwasserstoffreste,
- **a, b, c, d, f, g, h, i** und **k**: jeweils gleiche oder verschiedene ganze Zahlen im Wert von 0 bis 3, wobei die Summe **a+b+c+d+e+f+g+h+i+k** mindestens 2 und die Summe von **d** und **i** maximal 4 beträgt,
- **e**: eine Zahl im Wert von 0 oder 1,
- **o**: eine ganze Zahl im Wert von 0 bis 3,
- **p**: eine ganze Zahl im Wert von 0 bis 3 und einen durchschnittlichen Wert von 0,8 bis 2,2 und
- **q**: eine ganze Zahl im Wert von 0 bis 3 bedeuten und die Summe von **o, p** und **q** maximal 3 beträgt.

Die erfindungsgemäßen flüssigkristallinen Organosiloxane weisen eine deutlich größere HTP und damit ein besseres optisches Drehvermögen als die cholesterinhaltigen flüssigkristallinen Organosiloxane auf, so daß zur Erzielung der gleichen optischen Wirkung, beispielsweise zur Erzielung der gleichen Reflexionswellenlänge erheblich geringere Mengen an chiralen Weinsäureimidresten der allgemeinen Formel 1 als Dotierstoff eingesetzt werden müssen.

Die cholesterische Phase kann nach Orientierung oberhalb der Glastemperatur durch Abschrecken in den Glaszustand konserviert werden und ist bei Raumtemperatur stabil.

Vorzugsweise enthalten die flüssigkristallinen Organosiloxane neben den chiralen Weinsäureimidresten noch andere mesogene Reste, die die nachträgliche radikalische oder ionische Vernetzung ermöglichen.

Durch Verändern des Gehalts an chiralen Weinsäureimidresten und des Verhältnisses von chiralen Weinsäureimidresten zu anderen mesogenen Resten in den erfindungsgemäßen flüssigkristallinen Organosiloxanen kann die Reflexionswellenlänge der selektiven Reflexion eingestellt werden. Aufgrund der großen HTP-Werte benötigt man nur einen Anteil von 10 bis 20 Mol-%, bezogen auf alle in den Organosiloxanen vorhandenen mesogenen Reste an chiralen Weinsäureimidresten, um eine Reflexion im sichtbaren Bereich zu erhalten, während bei den entsprechenden cholesterinhaltigen Organosiloxanen zwischen 40 und 50 Mol-% Cholesterylreste benötigt werden.

Oftmals ist es aus anwendungstechnischen Gründen erwünscht, daß die mesogenen Gruppen der allgemeinen Formel 4 polare Funktionen, wie beispielsweise die Nitrilgruppe; enthalten, um im Flüssigkristall einen hohen dielektrischen Anisotropieeffekt zu erzielen.

Beispiele für unsubstituierte Reste R sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Oktylreste, wie der n-Octylrest und iso-Oktylreste, wie der 2,2,4-Trimethylpentylrest, Nonylreste, wie der n-Nonylrest, Decylreste, wie der n-Decylrest, Dodecylreste, wie der n-Dodecylrest, Oktadecylreste, wie der n-Oktadecylrest; Alkenylreste, wie der Vinyl- und der Allylrest; Cycloalkylreste, wie Cyclopentyl-, Cyclohexyl-, Cycloheptylreste und Methylcyclohexylreste; Arylreste, wie der Phenyl-, Naphthyl- und Anthryl- und Phenanthrylrest; Alkarylreste, wie o-, m-, p-Tolylreste, Xylylreste und Ethylphenylreste; Aralkylreste, wie der Benzylrest, der α- und der β-Phenylethylrest;

Beispiele für substituierte Reste **R** sind Cyanalkylreste, wie der β-Cyanethylrest, und halogenierte Kohlenwasserstoffreste, beispielsweise Halogenalkylreste, wie der 3,3,3-Trifluor-n-propylrest, der 2,2,2,2',2',2'-Hexafluorisopropylrest,
der Heptafluorisopropylrest, und Halogenarylreste, wie der o-, m-, und p-Chlorphenylrest. Vorzugsweise bedeutet R jeweils einen gegebenenfalls halogenierten Kohlenwasserstoffrest mit 1 bis 18, insbesondere 1 bis 10, Kohlenstoffatomen.

Besonders bevorzugt als Reste R sind C₁- bis C₄-Alkylreste und Phenylreste, insbesondere Methylreste.

Die Reste **X**^{**1**} und **X**^{**2**} können, falls sie nicht symmetrisch gebaut sind, mit jedem ihrer Enden an jedem ihrer Bindungspartner verbunden sein. So kann beispielsweise in den vorstehenden Formeln 3 und 4 und in den nachstehenden Formeln der Rest -COO- auch als -OOC-, der Rest -CONH- auch als -NHCO-, -CH=N- auch als -N=CH- gebunden sein.

Als Substituenten für die substituierten Arylene und Cycloalkylene **A**^{**1**}, **A**^{**2**}, **A**^{**3**} und **A**^{**4**} sind Halogenatome, C₁- bis C₄-Alkoxyreste, Nitro- und Cyanogruppen, C₁- bis C₆-Alkylreste, Carboxy-(C₁- bis C₄-Alkyl)-Reste und Tri-(C₁- bis C₄-Alkyl)-Siloxyreste bevorzugt.

Vorzugsweise hat in **R**^{**1**} **n** einen Wert von 3 bis 6 und vorzugsweise hat **m** den Wert 2n.

Beispiele für Reste **A**^{**5**} sind Alkylreste, wie der Methyl-, Ethyl-, n-Propyl-, iso-Propyl-, n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Oktylreste, wie der n-Octylrest und iso-Oktylreste, wie der 2,2,4-Trimethylpentylrest, Nonylreste, wie der n-Nonylrest, Decylreste, wie der n-Decylrest, Dodecylreste, wie der n-Dodecylrest, Hexadecylreste, wie der n-Hexadecylrest; Alkenylreste, wie der Vinyl- und der Allylrest, Butenyl-, Pentenyl-, Hexenyl-, Heptenyl-, Oktenyl-, Oktadienyl-, Decenyl-, Dodecenyl- und Hexadecenylreste; Cycloalkylreste, wie Cyclopentyl-, Cyclohexyl-, Cycloheptylreste und Methylcyclohexylreste; Alkyloxyreste, wie der Methoxy-, Ethoxy-, n-Propoxy-, Isopropoxy-, n-, sec.- und tert.-Butoxyrest, Pentoxy-, Hexoxy-, Oktoxy-, Decoxy-, Hexadecoxyreste; Alkenoxyreste, wie der Allyloxyrest, Butenyloxy-, Pentenyloxy-, Hexenyloxy-, Oktenyloxy-, Decenyloxy- und Hexadecenyloxyreste; Cycloalkylreste, wie der Cyclopentyl-, Cyclohexyl-, Cycloheptylrest; Cycloalkenylreste, wie Cyclopentenyl-, Cyclohexenyl- und Cycloheptenylreste; Cholestanreste; der Cholesterylrest; Fluor-, Chlor- oder Bromatome; Wasserstoffatome; Hydroxyl-, Nitril- und Trimethylsilyl-, Triethylsilylgruppen.

Es ist ganz besonders bevorzugt, daß -R¹-(X¹ₐ-A¹_{b}-A²_{c})_{d}- in der vorstehenden allgemeinen Formel 2 einen Rest der allgemeinen Formel 11, 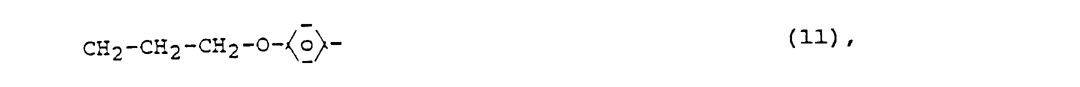 bedeutet.

Insbesondere bevorzugt als Reste der allgemeinen Formel 4 sind diejenigen der allgemeinen Formel 12, 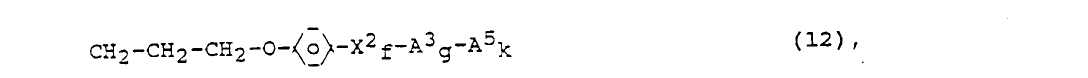 worin X², A³, A⁵, f, g und k die für die allgemeine Formel 4 angegebenen Bedeutungen haben und vorzugsweise f den Wert 1, g entweder 0 oder 1 und k den Wert 1 besitzen.

Es ist besonders bevorzugt, daß die chiralen Weinsäureimidreste die allgemeine Formel 13, 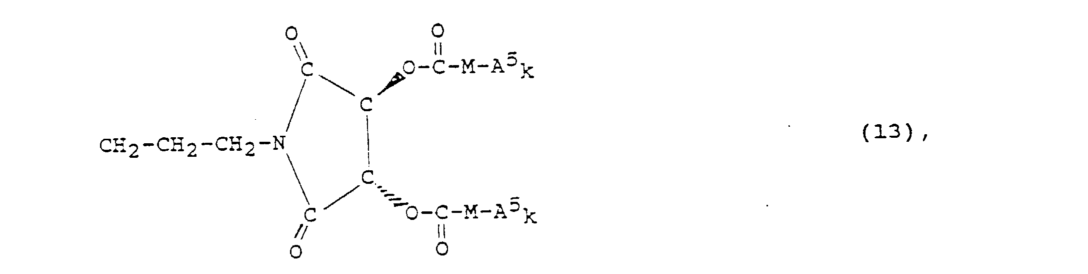 aufweisen, in der **M, A**^{**5**} und **k** die für die allgemeine Formel 1 angegebenen Bedeutungen besitzen.

Die erfindungsgemäßen flüssigkristallinen Organosiloxane können hergestellt werden durch Umsetzung von Organosiloxanen und/oder zu Organosiloxanen kondensierbaren Organosilanen mit mesogene Gruppen der allgemeinen Formel 1 und gegebenenfalls der allgemeinen Formel 4 aufweisenden Alkenen oder Alkinen, wobei die Organosiloxane und mindestens ein Teil der Organosilane mindestens ein direkt an Silicium gebundenes Wasserstoffatom aufweist.

In einem bevorzugten Verfahren zur Herstellung von flüssigkristallinen Organosiloxanen der vorstehenden allgemeinen Formel 3, bei denen n in den Resten der allgemeinen Formel 2 eine ganze Zahl im Wert von 2 bis 20 bedeutet, werden Organosiloxane, die aus Einheiten der allgemeinen Formel 14,

[RₚH_{q+o}SiO_{(4-o-p-q)/2}] (14),

aufgebaut sind, und/oder Organosilane der allgemeinen Formel 15

RᵣHₛ₊ₒSiY_{(4-o-r-s)/2} (15),

mit Verbindungen der allgemeinen Formel 16, 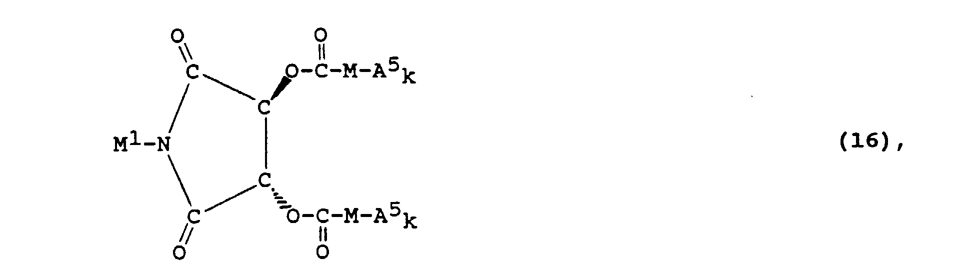 und gegebenenfalls Verbindungen der allgemeinen Formel 17,

R²-(X¹ ₐ-A¹ _{b}-A² _{c})_{d}-Zₑ-(-X² _{f}-A³ _{g}-A⁴ ₕ-)ᵢ-A⁵ ₖ (17),

umgesetzt,
und, falls Organosilane der allgemeinen Formel 15 eingesetzt werden, die erhaltenen Organosilane der allgemeinen Formel 18,

BₒRᵣHₛSiY_{(4-o-r-s)/2} (18),

kondensiert,
wobei in den vorstehenden Formeln 14 bis 18
- **M**^{**1**}: einen Rest der allgemeinen Formel 19,

R²-(X¹ ₐ-A¹ _{b}-A² _{c})_{d}-Zₑ-(-X² _{f}-A³ _{g}-A⁴ ₕ-)ᵢ (19),
- **Y**: eine kondensierbare Gruppe,
- **R**^{**2**}: einen Rest der Formel CₙHₘ¹, in der
- **m**^{**1**}: den Wert von 2n-1 oder 2n-3 besitzt, und
- **r** und **s**: jeweils eine ganze Zahl im Wert von 0 bis 3,bedeuten, die Summe von **o, r** und **s** maximal 3 beträgt, und **o, p, q, M, X**^{**1**}**, X**^{**2**}**, A**^{**1**}**, A**^{**2**}**, A**^{**3**}**, A**^{**4**}**, A**^{**5**}**, a, b, c, d, e, f, g, h, i, k, z, B** und **R** die Vorstehend angegebenen Bedeutungen aufweisen.

Vorzugsweise bedeutet **Y** ein Halogenatom oder eine C₁- bis C₄-Alkoxygruppe, insbesondere ein Chloratom oder eine Methoxy- oder Ethoxygruppe. Vorzugsweise beträgt der Wert von **s** 0 oder 1.

In den vorstehenden allgemeinen Formeln 16 und 17 hat in R² **n** vorzugsweise einen Wert von 3 bis 6, insbesondere 3 und vorzugsweise hat **m** den Wert 2n-1.

Besonders bevorzugte Verbindungen der allgemeinen Formel 17 sind diejenigen der allgemeinen Formel 20, 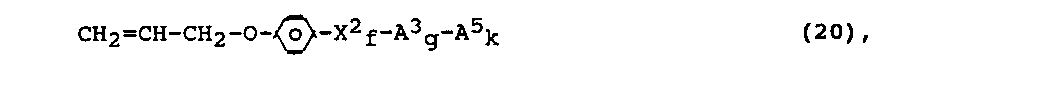 worin **X**^{**2**}**, A**^{**3**}**, A**^{**5**}**, f, g** und **k** die für die allgemeine Formel 4 angegebenen Bedeutungen haben und vorzugsweise **f** den Wert 1, **g** entweder 0 oder 1 und k den Wert 1 besitzen.

Besonders bevorzugte Verbindungen der allgemeinen Formel 16 sind diejenigen der allgemeinen Formel 21, 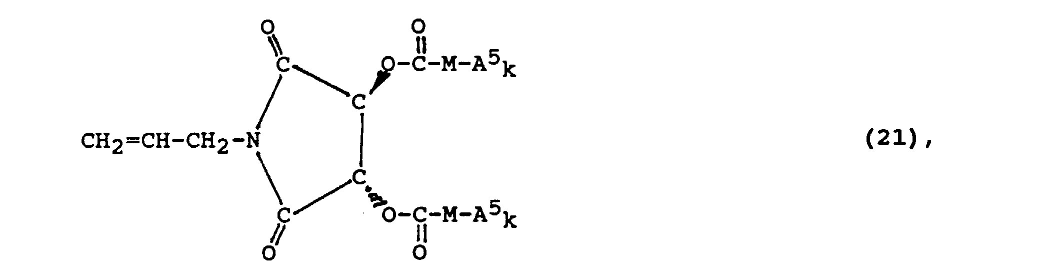 in der **M, A**^{**5**} und **k** die für die allgemeine Formel 1 angegebenen Bedeutungen besitzen.

Die Verbindungen der allgemeinen Formel 21 lassen sich an direkt an Silicium gebundene Wasserstoffatome hydrosilylieren, wobei kaum unerwünschte Nebenreaktionen auftreten. Entsprechende Allyloxyester neigen sonst in hohem Maße zur Isomerisierung der Doppelbindung und anschließender Propenabspaltung. Dies ist beispielsweise in der EP-A- 466 183 beschrieben.

Bevorzugt werden als Siloxane der allgemeinen Formel (14) solche eingesetzt, die zu mindestens 90 % ihrer Einheiten aus solchen der Formeln 5 bis 10,

[(CH₃)₂SiO] (5), [(CH₃)HSiO] (6), [H₂SiO] (7), [H(CH₃)₂SiO_{1/2}] (8), [(CH₃)₃SiO_{1/2}] (9) und [HSiO_{3/2}] (10),

aufgebaut sind und 2 bis 100 Siliciumatome pro Molekül, insbesondere 2 bis 15 Siliciumatome pro Molekül, enthalten. Besonders bevorzugt sind cyclische Siloxane, die aus Einheiten der Formeln 6 und gegebenenfalls 5 aufgebaut sind.

Die Umsetzung von direkt an Silicium gebundene Wasserstoffatome aufweisenden Organosiloxanen und/oder zu Organosiloxanen kondensierbaren Organosilanen mit Alkenen oder Alkinen der allgemeinen Formeln 16 und 17 erfolgt in an sich bekannter Weise, z.B. durch Hydrosilylierung in Lösungsmitteln, wie Kohlenwasserstoffen, Ethern oder Estern mit Metallen oder Verbindungen der Platingruppe als Katalysator. Geeignete Verfahren zur Hydrosilylierung sind beispielsweise in der EP-A-466 183 beschrieben.

Zur Herstellung von erfindungsgemäßen flüssigkristallinen Organosiloxanen, welche in den mesogenen Resten der allgemeinen Formel 4 Methacryloxy- und /oder Acryloxygruppen aufweisen, ist das in der EP-A-358 208 beschriebene Verfahren bevorzugt.

Vorzugsweise werden 0,1 bis 10 Mol, insbesondere 0,5 bis 2 Mol, von Verbindungen der Formeln 3 und 4 pro Grammatom direkt an Siliciumatome gebundene Wasserstoffatome bei der Hydrosilylierung eingesetzt.

Werden im vorstehend geschilderten Verfahren Organosilane, beispielsweise der allgemeinen Formel 15, eingesetzt, so werden diese gemeinsam mit chirale Weinsäureimidreste der allgemeinen Formel 1 enthaltenden Organosilanen oder Organosiloxanen nach an sich bekannten Verfahren zu flüssigkristallinen Organosiloxanen kondensiert. Dies kann u.a. durch Umsetzung mit Säuren, wie wäßriger Salzsäure, erfolgen. Derartige Verfahren sind beschrieben in W.Noll: Chemistry and Technology of Silicones, Academic Press, Orlando Fla., 1968, Seite 191 bis 239.

Durch die vorstehend beschriebenen Reaktionen erhält man ein Gemisch unterschiedlicher Moleküle.

Die Erfindung betrifft auch die Weinsäurealkenimide der vorstehenden allgemeinen Formel 16, die Zwischenprodukte bei der Herstellung der flüssigkristallinen Organosiloxane sind.

Die Synthese der Weinsäurealkenimide kann beispielsweise nach folgenden Verfahren erfolgen:
1.) Im ersten Schritt wird ein chirales Weinsäureanhydrid durch Reaktion von drei äquivalenten Säurechlorid mit Weinsäure nach der Beschreibung von JACS 1933, 55, 2605 hergestellt und anschließend mit Alkenaminen bei Temperaturen zwischen 120 und 160°C in geeigneten inerten Lösemitteln, wie Toluol umgesetzt.
2.) Im ersten Schritt wird ein Weinsäurealkenimid durch Erhitzen von Weinsäure in Gegenwart von stöchiometrischen Mengen Alkenamin hergestellt. Anschließend werden die beiden noch freien OH-Gruppen mit Carbonsäuren oder Carbonsäurechloriden nach an sich bekannten Verfahren verestert. Dieses Verfahren ist in der US-A-4,996,330 beschrieben.

Die neuen Organosilane der vorstehenden allgemeinen Formel 18, bei denen **o** eine ganze Zahl im Wert von 1, 2 oder 3 bedeutet, sind als Zwischenprodukte zur Herstellung der flüssigkristallinen Organosiloxane ebenfalls Gegenstand der vorliegenden Erfindung.

Die erfindungsgemäßen flüssigkristallinen Organosiloxane können auf verschiedene Weise in optischen Elementen, für dekorative Zwecke und als polarisierende Farbfilter, insbesondere Notch-Filter, verwendet werden. Sie erlauben es, den rechtshändig oder linkshändig polarisierten Anteil des Lichts in bestimmten vorgegebenen Spektralbereichen zu reflektieren.

Für die vorstehende Anwendung können sowohl Mischungen der erfindungsgemäßen Organosiloxane untereinander als auch Mischungen der erfindungsgemäßen Organosiloxane mit anderen flüssigkristallinen Materialien oder reine Weinsäureimidreste enthaltende Organosiloxane genutzt werden. Insbesondere können auch Mischungen mit anderen flüssigkristallinen Substanzen verwendet werden, wodurch eine Abstimmung der Reflexionswellenlänge zwischen 400 nm rechtshelikal über infrarot rechtshelikal, nematisch (= unendlicher Pitch), infrarot linkshelikal bis 400 nm linkshelikal erfolgen kann.

Die Mischungen der flüssigkristallinen Organosiloxane untereinander und mit anderen flüssigkristallinen Materialien sind ebenfalls Gegenstand der vorliegenden Erfindung. Weitere Bestandteile derartiger Gemische können beispielsweise monomere flüssigkristalline Methacrylsäure-und/oder Acrylsäureester sein. Die flüssigkristallinen Mischungen aus methacryloxy- und/oder acryloxygruppen aufweisenden Komponenten können nach an sich bekannten Verfahren polymerisiert und bei geeigneter Wahl der Komponenten auch vernetzt werden.

Die erfindungsgemäßen flüssigkristallinen Organosiloxane, welche in den mesogenen Resten der allgemeinen Formel 4 Methacryloxy- und/oder Acryloxygruppen aufweisen, können dreidimensional vernetzt werden. Diese Vernetzung wird vorzugsweise mittels freier Radikale bewirkt, welche durch Peroxide, durch UV-Licht oder durch energiereichere elektromagnetische Strahlung als UV-Licht, oder thermisch erzeugt werden. Die Vernetzung kann aber auch mittels direkt an Siliciumatome gebundene Wasserstoffatome enthaltende Vernetzer unter Katalyse durch die oben genannten Platinmetallkatalysatoren bewirkt werden. Sie kann auch kationisch oder anionisch erfolgen. Besonders bevorzugt ist die Vernetzung durch UV-Licht. Diese Vernetzung ist in der EP-A-358 208 beschrieben.

In den nachfolgenden Beispielen sind, falls jeweils nicht anders angegeben,
a) alle Mengenangaben auf das Gewicht bezogen;
b) alle Drücke 0,10 MPa (abs.);
c) alle Temperaturen 20°C;
d) HTP = helical twisting power;

### Herstellung der Weinsäurealkenimide:

Die nachstehenden Weinsäurealkenimide la bis ld wurden nach den vorstehend beschriebenen Verfahren 1 und 2 hergestellt und mit anderen olefinhaltigen Mesogenen zu flüssigkristallinen Organosiloxanen verarbeitet. 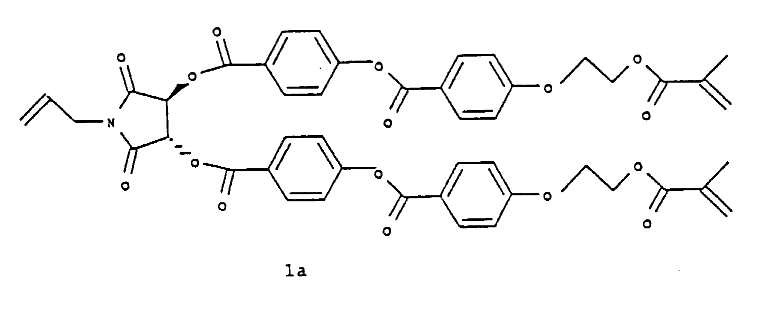 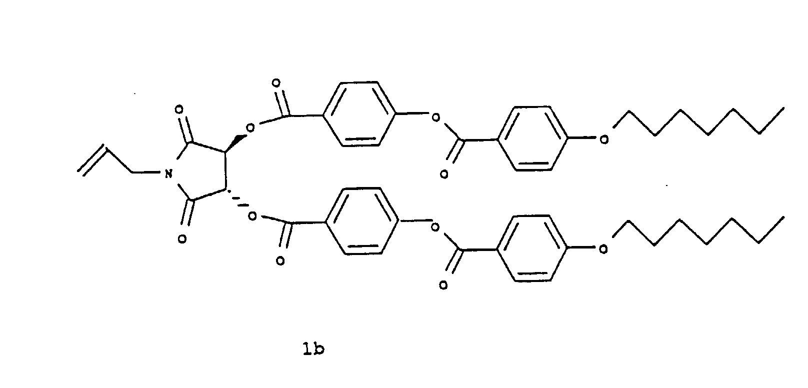 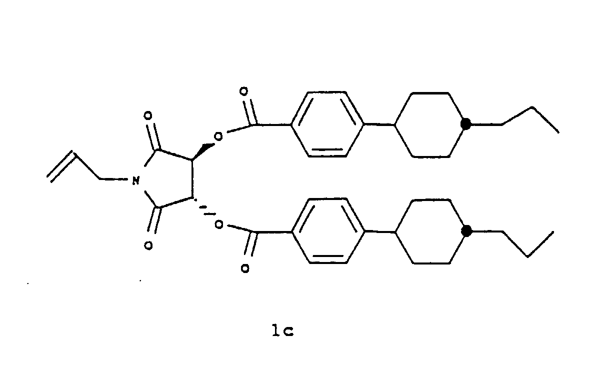 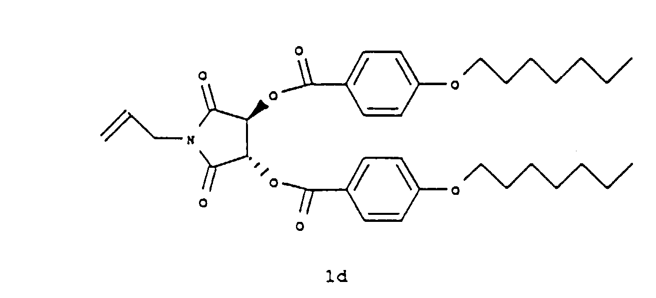 Herstellung von (3R, 4R)-(+)-Allyl-3,4-bis-[4-(trans-n-propylcyclohexyl)-benzoyloxy]-succinimid (Weinsäurealkenimid 1-c) nach dem vorstehend beschriebenen Verfahren 2

### a) Herstellung des Allylimides

200 g (1,33 mol) L(+)-Weinsäure und 76 g (1,33 mol) Allylamin wurden in 70 ml heißem Wasser aufgelöst und ca. 9 Stunden auf 140°C erhitzt. Dabei entstehendes Wasser wurde abdestilliert. Das stark gefärbte Produkt wurde aus Ethanol umkristallisiert. Es wurden 48,4 g (21% d. Th.) farblose Kristalle erhalten.

### b) Veresterung des Allylimides

24,6 g (0,10 mol) (4-trans-n-propylcyclohexyl)-4-benzoesäure, 17,5 g (0,15 mol) Thionylchlorid und einige Tropfen DMF wurden 3,5 Stunden lang bei 60°C in 50 ml Toluol gerührt. Dann wurden das überschüssige Thionylchlorid und das Toluol im Vakuum abdestilliert.

Das so erhaltene Säurechlorid wurde in 50 ml Toluol aufgenommen und zu einer Mischung aus 8,56 g (0,05 mol) Allylimid, 15,2 g (0,15 mol) Triethylamin, einer Spatelspitze 4-Dimethylaminopyridin und 150 ml Toluol bei 10°C zugetropft.

Nach 18 Stunden rühren wurde das Reaktionsgemisch zunächst mit 1 n HCl, dann mit NaHCO₃-Lösung und H₂O augeschüttelt. Nach dem Trocknen wurde das Lösemittel abgedampft. Es wurden 25,7 g (82 % d. Th.) Produkt erhalten.

### Beispiel 1:

1,0 g (1,6 mmol)
(3R,4R)-(+)-Allyl-3,4-bis-[4-(trans-n-propyl-cyclohexyl)-benzoyloxy]-succinimid (Weinsäurealkenimid 1c), 3,2 g (8,0 mmol) 4-(Propen-2-oxy)benzoesäure-4(4-methoxyphenyl)phenylester und 0,96 g (3,2 mmol) Pentamethylcyclopentasiloxan wurden unter leichtem Erwärmen in 40ml Toluol gelöst. Nach Zugabe von 0,1 ml Katalysatorlösung (1 Gew.% Dicyclopentadienplatindichlorid in Methylenchlorid) wurde die Lösung eine Stunde bei 100°C gerührt. Zur auf unter 50°C abgekühlten Lösung gab man 10 mg Aluminiumsalz von N-Nitrosophenylhydroxylamin und 2,16 g (6,4 mmol) 4-(Propen-2-oxy)benzoesäure-4-methacryloxyphenylester gelöst in 20 ml Toluol und rührt eine Stunde bei 70°C. Das so erhaltene Rohprodukt wurde zur Abtrennung des Katalysators über eine kurze mit Kieselgel gefüllte Säule (1=3cm,d=3cm) filtriert und einrotiert. Man erhielt 4,76g (65%d.Th.) einer Substanz mit einer Reflexionswellenlänge von 660 nm; das Material besaß einen Glaspunkt von -14°C und eine cholesterische Phase bis zum Klärpunkt von 145°C und hatte die folgende Formel

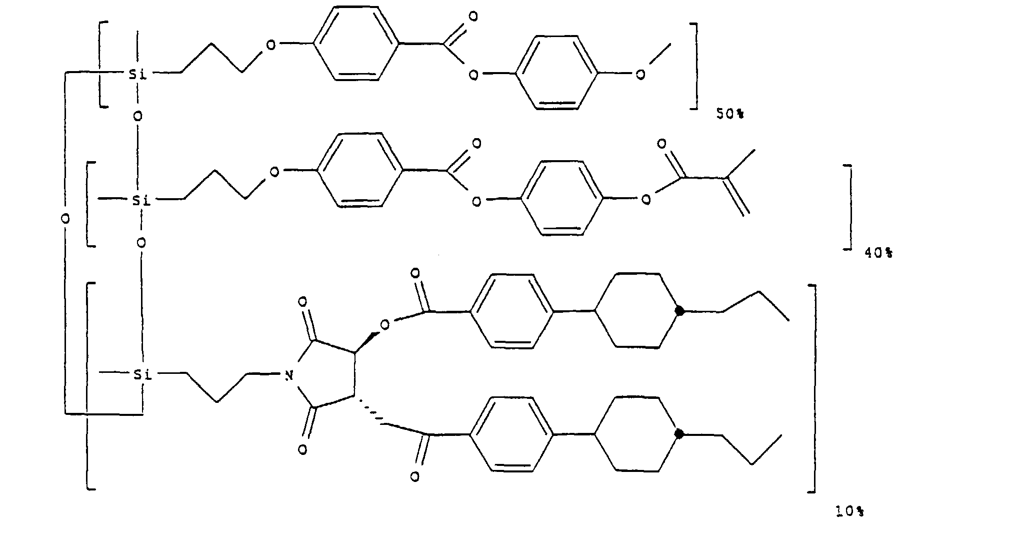

### Vergleichsbeispiel 1

analog Beispiel 1 wurde folgendes flüssigkristallines Polyorganosiloxan mit Cholesterin als Chiralikum hergestellt.

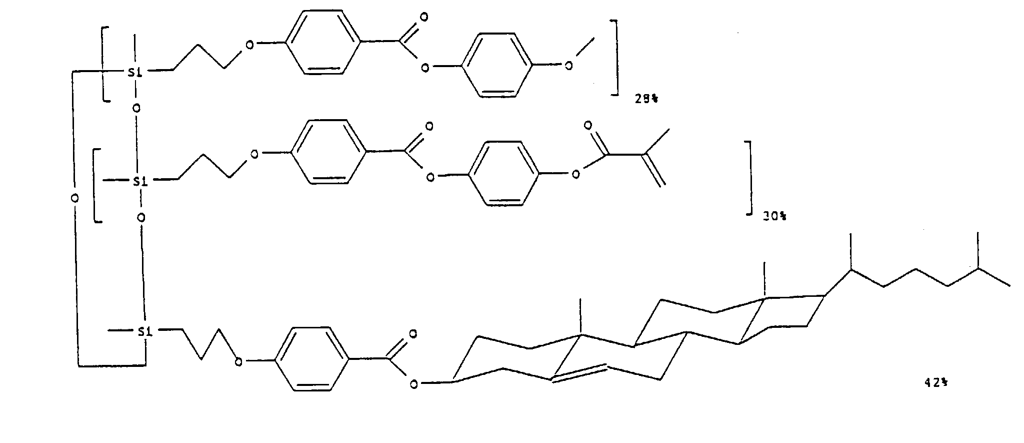

Die Reflexionswellenlänge liegt auch hier bei 660 nm. Es wird hier aber mehr als die vierfache Menge an Chiralikum benötigt, um diese zu erreichen.

### Beispiel 2:

1,0 g (1,6 mmol)
(3R,4R)-(+)-Allyl-3,4-bis-[4-(trans-n-propyl-cyclohexyl)-benzoyloxy]-succinimid (Weinsäurealkenimid 1c), 1,93 g (4,8 mmol) 4-(Propen-2-oxy)benzoesäure-4(4-methoxyphenyl)phenylester und 0,64 g (2,1 mmol) Pentamethylcyclopentasiloxan wurden unter leichtem Erwärmen in 40ml Toluol gelöst. Nach Zugabe von 0,1 ml Katalysatorlösung (1 Gew.% Dicyclopentadienplatindichlorid in Methylenchlorid) wurde die Lösung eine Stunde bei 100°C gerührt. Zur auf unter 50°C abgekühlten Lösung gab man 10 mg Aluminiumsalz von N-Nitrosophenylhydroxylamin und 1,44 g (4,25 mmol) 4-(Propen-2-oxy)benzoesäure-4-methacryloxyphenylester gelöst in 20 ml Toluol und rührte eine Stunde bei 70°C. Das so erhaltene Rohprodukt wurde zur Abtrennung des Katalysators über eine kurze mit Kieselgel gefüllte Säule (l=3cm,d=3cm) filtriert und einrotiert. Man erhielt 3,1g (62%) einer Substanz mit einer Reflexionsionswellenlänge von 540 nm; das Material besaß einen Glaspunkt von -20°C und eine cholesterische Phase bis zum Klärpunkt von 145°C.

### Beispiel 3:

0,5 g (0,8 mmol)
(3R,4R)-(+)-Allyl-3,4-bis-[4-(trans-n-propyl-cyclohexyl)-benzoyloxy]-succinimid (Weinsäurealkenimid 1c), 1,6 g (4,0 mmol) 4-(Propen-2-oxy)benzoesäure-4(4-methoxyphenyl)phenylester, 0,9 g (3,2 mmol) 4-(Propen-2-oxy)-4-ethoxyazobenzol und 0,48 g (1,6 mmol) Pentamethylcyclopentasiloxan wurden unter leichtem Erwärmen in 30 ml Toluol gelöst. Nach Zugabe von 0,1 ml Katalysatorlösung (1 Gew.% Dicyclopentadienplatindichlorid in Methylenchlorid) wurde die Lösung drei Stunden bei 100°C gerührt. Das so erhaltene Rohprodukt wurde zur Abtrennung des Katalysators über eine kurze mit Kieselgel gefüllte Säule (l=3cm,d=3cm) filtriert und zweimal aus Toluol in Ethanol umgefällt. Man erhielt 2,1g (60%) einer Substanz mit einer Reflexionswellenlänge von 840 nm; das Material besaß einen Glaspunkt von 0°C und eine cholesterische Phase bis zum Klärpunkt von 120°C.

### Beispiel 4:

870 mg (1.39 mmol) (3R,4R)-(+)-Allyl-3,4-bis-[4-(trans-n-propyl-cyclohexyl)-benzoyloxy]-succinimid, 1.48g (5.2mmol) 4-(Propen-2-oxy)benzoesäure-4(4-methoxyphenyl)phenylester, 2.5g (6.6 mmol) 4-(Propen-2-oxy)benzoesäure-[4-(4-trans-n-propyl-cyclohexyl) -phenylester] und 833 mg (2.77mmol) Pentamethylcyclopentasiloxan wurden unter leichtem Erwärmen in 15ml Toluol gelöst. Nach Zugabe von 0.05 ml Katalysatorlösung (0.5 Gew. % Dicyclopentadienplatindichlorid in Methylenchlorid) wurde die Lösung 4 Stunden am Rückfluß erhitzt. Anschließend wurde die toluolische Lösung des Reaktionsproduktes zur weiteren Reinigung in Petrolether getropft. Man erhielt 4.5g (80%d.Th.) einer Substanz mit der Reflexionswellenlänge von 610 nm; das Material hatte einen Glaspunkt von 9°C und eine cholesterische Phase bis zum Klärpunkt von 125°C und besaß folgende Formel

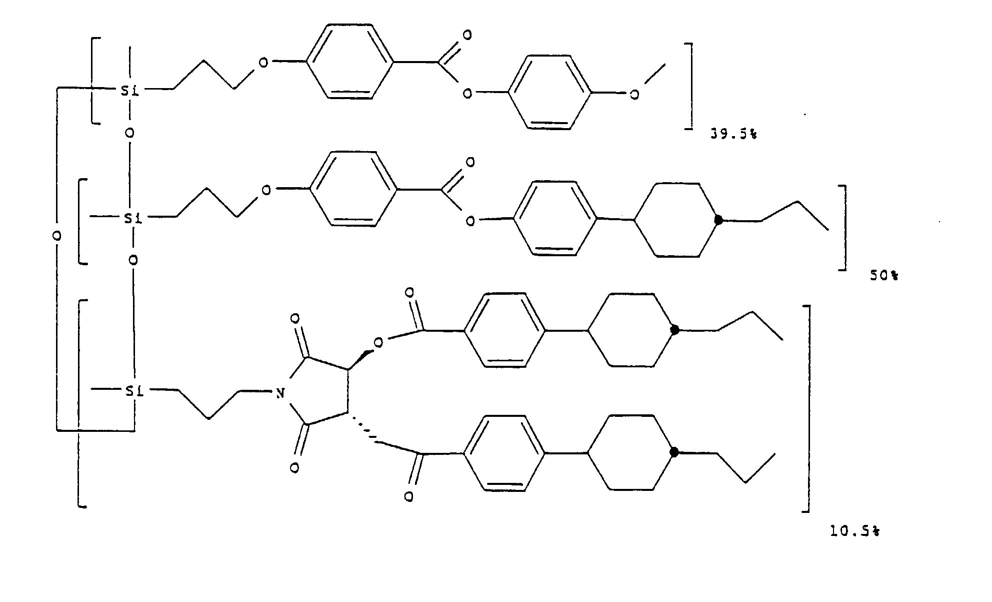

## Patentansprüche

1. Flüssigkristalline Organosiloxane, welche pro Molekül mindestens einen Si-C gebundenen chiralen Weinsäureimidrest der allgemeinen Formel (1) 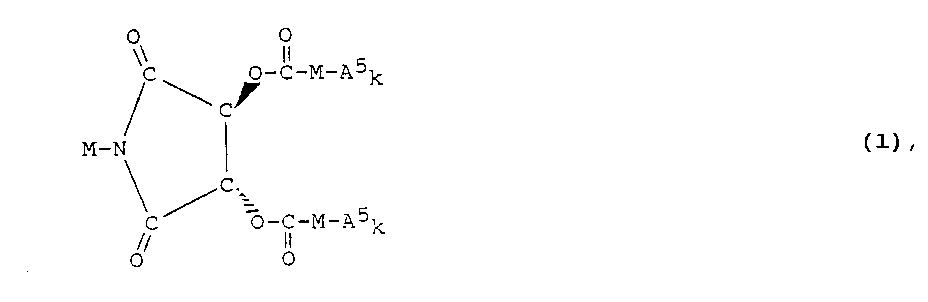 aufweisen, in der
**M** einen Rest der allgemeinen Formel 2,
R¹-(X¹ ₐ-A¹ _{b}-A² _{c})_{d}-Zₑ-(-X² _{f}-A³ _{g}-A⁴ ₕ-)ᵢ (2),
bedeutet,
wobei die Organosiloxane aus mindestens zwei Einheiten der allgemeinen Formel 3,
[BₒRₚH_{q}SiO_{(4-o-p-q)/2}] (3),
aufgebaut sind, in der
**B** einen Rest der allgemeinen Formel 1, und gegebenenfalls einen Rest der allgemeinen Formel 4,
M-A⁵ ₖ (4),
bedeutet, wobei in den vorstehenden allgemeinen Formeln 1 bis 4
**R**^{**1**} einen Rest der Formel CₙHₘ bedeutet, in der
**n** eine ganze Zahl im Wert von 0 bis 20,
**m** den Wert von 2n besitzt, oder, falls n mindestens 2 bedeutet, auch den Wert von (2n-2) besitzen kann, und in R¹ eine oder mehrere Methyleneinheiten durch Sauerstoffatome ersetzt sein können, welche an Kohlenstoff- und/oder Siliciumatome gebunden sein können,
**X**^{**1**} und **X**^{**2**} gleiche oder verschiedene zweibindige Reste aus der Gruppe -O-, -COO-, -CONH-, -CO-, -S-, -C≡C-, -CH=CH-, -CH=N-, -CH₂-, -N=N- und -N=N(O)-,
**A**^{**1**}**, A**^{**2**}**, A**^{**3**} und **A**^{**4**} gleiche oder verschiedene zweibindige Reste, nämlich 1,4-Phenylen-, 1,4-Cyclohexylenreste, substituierte Arylene mit 1 bis 10 Kohlenstoffatomen, substituierte Cycloalkylene mit bis 10 Kohlenstoffatomen und Heteroarylene mit 1 bis 10 Kohlenstoffatomen,
**Z** gleiche oder verschiedene zwei- bis vierwertige Benzol-, Cyclohexan- oder Cyclopentanreste,
**A**^{**5**} gleiche oder verschiedene, gesättigte oder olefinisch ungesättigte Alkyl-, Alkoxy- oder Cycloalkylreste mit jeweils 1 bis 16 Kohlenstoffatomen, Cholestanreste, Cholesterylreste, Halogenatome, Wasserstoffatome, Hydroxyl-, Nitril-, Acryloxy-, (Meth)acryloxy-, (Meth)acryloxyethylenoxy-, (Meth)acryloxydi(ethylenoxy)-, (Meth)acryloxytri(ethylenoxy)-, R- oder S-Tetrahydrofurancarbonsäureester und Trialkylsiloxygruppen, deren Alkylreste jeweils 1 bis 8 Kohlenstoffatome besitzen,
**R** gleiche oder verschiedene, gegebenenfalls substituierte C₁- bis C₁₈-Kohlenwasserstoffreste,
**a, b, c, d, f, g, h, i** und **k** jeweils gleiche oder verschiedene ganze Zahlen im Wert von 0 bis 3, wobei die Summe **a+b+c+d+e+f+g+h+i+k** mindestens 2 und die Summe von **d** und **i** maximal 4 beträgt,
**e** eine Zahl im Wert von 0 oder 1,
**o** eine ganze Zahl im Wert von 0 bis 3,
**p** eine ganze Zahl im Wert von 0 bis 3 und einen durchschnittlichen Wert von 0,8 bis 2,2 und
**q** eine ganze Zahl im Wert von 0 bis 3 bedeuten und die Summe von **o, p** und **q** maximal 3 beträgt.

2. Verfahren zur Herstellung von flüssigkristallinen Organosiloxanen gemäß Anspruch 1, bei denen n in den Resten der allgemeinen Formel 2 eine ganze Zahl im Wert von 2 bis 20 bedeutet, dadurch gekennzeichnet, daß Organosiloxane, die aus Einheiten der allgemeinen Formel 14,
[RₚH_{q+o}SiO_{(4-o-p-q)/2}] (14),
aufgebaut sind, und/oder Organosilane der allgemeinen Formel 15
RᵣHₛ₊ₒSiY_{(4-o-r-s)/2} (15),
mit Verbindungen der allgemeinen Formel 16, 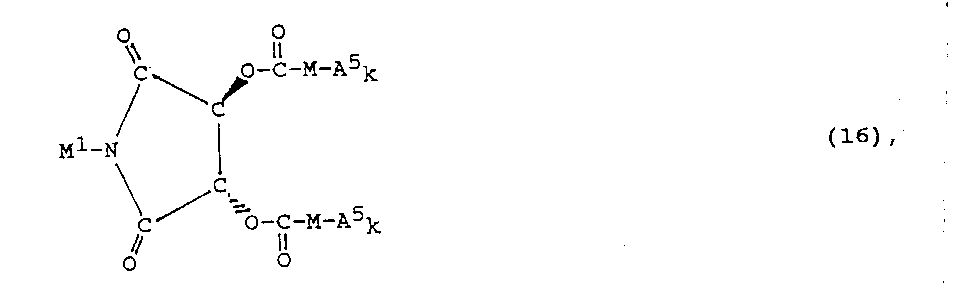 und gegebenenfalls Verbindungen der allgemeinen Formel 17,
R²-(X¹ ₐ-A¹ _{b}-A² _{c})_{d}-Zₑ-(-X² _{f}-A³ _{g}-A⁴ ₕ-)ᵢ-A⁵ ₖ (17),
umgesetzt werden,
und, falls Organosilane der allgemeinen Formel 15 eingesetzt werden, die erhaltenen Organosilane der allgemeinen Formel 18,
BₒRᵣHₛSiY_{(4-o-r-s)/2} (18),
kondensiert werden,
wobei in den vorstehenden Formeln 14 bis 18
**M**^{**1**} einen Rest der allgemeinen Formel 19,
R²-(X¹ ₐ-A¹ _{b}-A² _{c})_{d}-Zₑ-(-X² _{f}-A³ _{g}-A⁴ ₕ-)ᵢ (19),
**Y** eine kondensierbare Gruppe,
**R**^{**2**} einen Rest der Formel CₙHₙ¹, in der
**m**^{**1**} den Wert von 2n-1 oder 2n-3 besitzt, und
r und **s** jeweils eine ganze Zahl im Wert von 0 bis 3, bedeuten, die Summe von **o, r** und **s** maximal 3 beträgt, und **o, p, q, M, X**^{**1**}**, X**^{**2**}**, A**^{**1**}**, A**^{**2**}**, A**^{**3**}**, A**^{**4**}**, A**^{**5**}**, a, b, c, d,** **e, f, g, h, i, k, Z, B** und **R** die in Anspruich 1 angegebenen Bedeutungen aufweisen.

3. Mischungen der flüssigkristallinen Organosiloxane gemäß Anspruch 1 untereinander und mit anderen flüssigkristallinen Materialien.

4. Verwendung der flüssigkristallinen Organosiloxane gemäß Anspruch 1 oder der Mischungen gemäß Anspruch 3 als chirale Filtermaterialien, für dekorative Zwecke und in optischen Elementen.

5. Verbindungen der allgemeinen Formel 16, 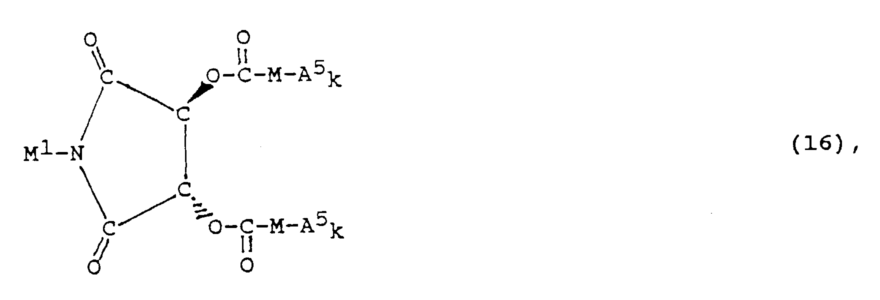 in der **M, M**^{**1**}**, A**^{**5**} und **k** die in den Ansprüchen 1 und 2 angegebenen Bedeutungen aufweisen.

6. Organosilane der allgemeinen Formel 18,
BₒRᵣHₛSiY_{(4-o-r-s)/2} (18),
bei denen **o** eine ganze Zahl im Wert von 1, 2 oder 3 bedeutet und **B, R, Y, r** und **s** die in den Ansprüchen 1 und 2 angegebenen Bedeutungen aufweisen.

## Claims

1. Liquid-crystalline organosiloxane containing, per molecule, at least one Si-C-bonded chiral tartarimide radical of the general formula (1) 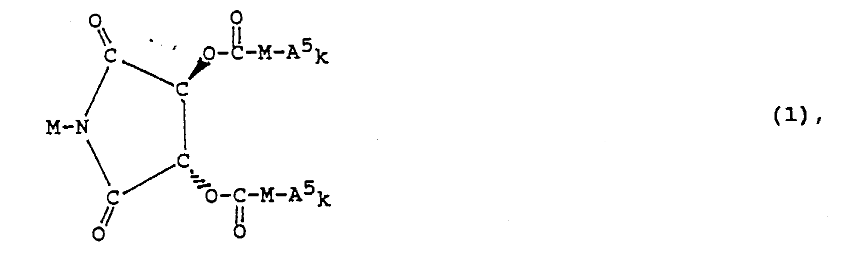 in which
**M** is a radical of the general formula 2,
R¹-(X¹ ₐ-A¹ _{b}-A² _{c})_{d}-Zₑ- (-X² _{f}-A³ _{g}-A⁴ ₕ-)ᵢ (2),
where the organosiloxanes are built up from at least two units of the general formula 3
[BₒRₚH_{q}Sio_{(4-o-p-q)/2}] (3),
in which
**B** is a radical of the general formula 1, and, if desired, a radical of the general formula 4,
M-A⁵ ₖ (4),
where, in the above general formulae 1 to 4,
**R**^{**1**} is a radical of the formula CₙHₘ, in which
**n** is an integer having a value of from 0 to 20,
**m** has the value 2n or, if n is at least 2, can also have the value (2n-2), and one or more methylene units in R¹ can be replaced by oxygen atoms, which can be bonded to carbon and/or silicon atoms,
**X**^{**1**} and **X**^{**2**} are identical or different divalent radicals from the group consisting of -O-, -COO-, -CONH-, -CO-, -S-, -C≡C-, -CH=CH-, -CH=N-, -CH₂-, -N=N- and -N=N(O)-,
**A**^{**1**}**, A**^{**2**}**, A**^{**3**} and **A**^{**4**} are identical or different divalent radicals, namely 1,4-phenylene, 1,4-cyclohexylene, substituted arylenes having 1 to 10 carbon atoms, substituted cycloalkylenes having 1 to 10 carbon atoms and heteroarylenes having 1 to 10 carbon atoms,
**Z** are identical or different divalent to tetravalent benzene, cyclohexane or cyclopentane radicals,
**A**^{**5**} are identical or different, saturated or olefinically unsaturated alkyl, alkoxy or cycloalkyl radicals, each having 1 to 16 carbon atoms, cholestane radicals, cholesteryl radicals, halogen atoms, hydrogen atoms, hydroxyl, nitrile, acryloxy, (meth)acryloxy, (meth)acryloxyethyleneoxy, (meth)acryloxydi(ethyleneoxy), (meth)acryloxytri(ethyleneoxy), R- or S-tetrahydrofurancarboxylate and trialkylsiloxy groups whose alkyl radicals each have 1 to 8 carbon atoms,
**R** are identical or different, substituted or unsubstituted C₁- to C₁₈-hydrocarbon radicals,
**a, b, c, d, f, g, h, i** and **k** are each identical or different integers having a value of from 0 to 3, where the sum **a+b+c+d+e+f+g+h+i+k** is at least 2 and the sum of **d** and **i** is at most 4,
**e** is a number having a value of 0 or 1,
**o** is an integer having a value of from 0 to 3,
**p** is an integer having a value of from 0 to 3 and a mean value of from 0.8 to 2.2,
**q** is an integer having a value of from 0 to 3, and the sum of **o, p** and **q** is at most 3.

2. Process for the preparation of the liquid-crystalline organosiloxanes according to Claim 1, in which n in the radicals of the general formula 2 is an integer having a value of from 2 to 20, characterized in that organosiloxanes built up from units of the general formula 14,
[RₚH_{q+o}SiO_{(4-o-p-q)/2}] (14),
and/or organosilanes of the general formula 15
RᵣHₛ₊ₒSiY_{(4-o-r-s)/2} (15),
are reacted with compounds of the general formula 16 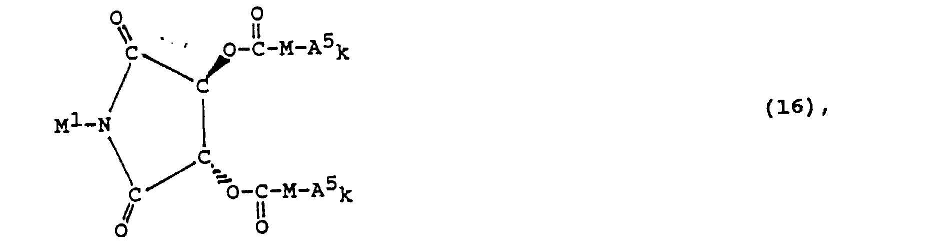 and, if desired, compounds of the general formula 17
R²-(X¹ ₐ-A¹ _{b}-A² _{c})_{d}-Zₑ-(-X² _{f}-A³ _{g}-A⁴ ₕ-)ᵢ-A⁵ ₖ (17),
and, if organosilanes of the general formula 15 are employed, the resultant organosilanes of the general formula 18
BₒRᵣHₛSiY_{(4-o-r-s)/2} (18),
are condensed,
where, in the above formulae 14 to 18,
**M**^{**1**} is a radical of the general formula 19
R²-(X¹ ₐ-A¹ _{b}-A² _{c})_{d}-Zₑ-(-X² _{f}-A³ _{g}-A⁴ ₕ-)ᵢ (19),
**Y** is a condensable group,
**R**^{**2**} is a radical of the formula CₙHₘ¹, in which
**m**^{**1**} has the value 2n-1 or 2n-3, and
**r** and **s** are each an integer having a value of from 0 to 3, the sum of **o, r** and **s** is at most 3, and **o, p, q, M, X**^{**1**}**, X**^{**2**}**, A**^{**1**}**, A**^{**2**}**, A**^{**3**}**, A**^{**4**}**, A**^{**5**}**, a, b, c, d, e, f, g, h, i, k, z, B** and **R** are as defined in Claim 1.

3. Mixtures of liquid-crystalline organosiloxanes according to Claim 1 with one another or with other liquid-crystalline materials.

4. Use of the liquid-crystalline organosiloxanes according to Claim 1 or a mixture according to Claim 3 as chiral filter materials, for decorative purposes or in optical elements.

5. Compounds of the general formula 16 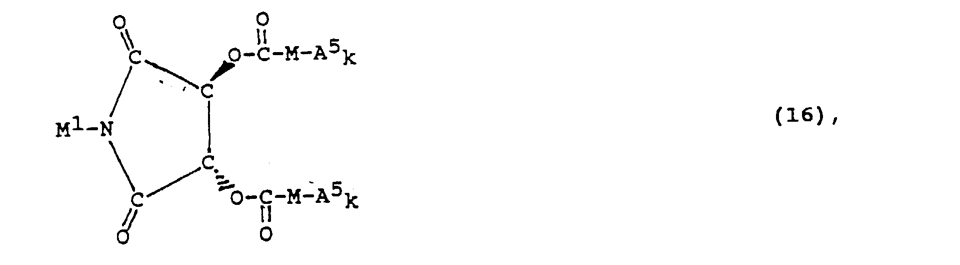 in which **M, M**^{**1**}**, A**^{**5**} and **k** are as defined in Claims 1 and 2.

6. Organosilanes of the general formula 18,
BₒRᵣHₛSiY_{(4-o-r-s)/2} (18),
in which **o** is an integer having a value of 1, 2 or 3, and **B, R, Y, r** and **s** are as defined in Claims 1 and 2.

## Revendications

1. Organosiloxanes cristallins liquides contenant, par molécule, au moins un radical tartarimide chiral lié à Si-C de formule générale (1) 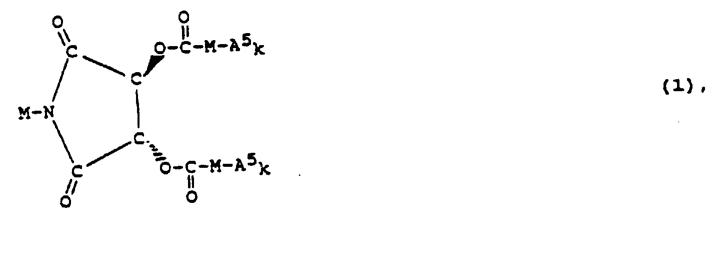 dans laquelle
M représente un radical de formule générale 2,
R¹-(X¹ ₐ-A¹ _{b}-A² _{c})_{d}-Zₑ-(-X² _{f}-A³ _{g}-A⁴ ₕ-)ᵢ (2),
les organosiloxanes étant composés d'au moins deux motifs de formule générale 3,
[BₒRₚH_{q}SiO_{(4-o-p-q)/2}] (3),
dans laquelle
B représente un radical de formule générale 1, et éventuellement un radical de formule générale 4,
M-A⁵ ₖ (4),
où, dans les formules générales 1 à 4 ci-dessus,
R¹ représente un radical de formule CₙHₘ, dans laquelle
n représente un entier ayant une valeur de 0 à 20,
m a la valeur 2n, ou si n vaut au moins 2, peut également avoir la valeur (2n-2), et un ou plusieurs motifs méthylène dans R¹ peuvent être remplacés par des atomes d'oxygène, qui peuvent être liés à des atomes de carbone et/ou de silicium,
X¹ et X² représentent des radicaux bivalents, identiques ou différents, parmi le groupe constitué de -O-, -COO-, -CONH-, -CO-, -S-, -C≡C-, -CH=CH-, -CH=N-, -CH₂-, -N=N- et -N=N(O)-,
A¹, A², A³ et A⁴ représentent des radicaux bivalents, identiques ou différents, tels que les radicaux 1,4-phénylène, 1,4-cyclohexylène, des arylènes substitués ayant 1 à 10 atomes de carbone, des cycloalkylènes substitués ayant 1 à 10 atomes de carbone et des hétéroarylènes ayant 1 à 10 atomes de carbone,
Z représente des radicaux benzène, cyclohexane ou cyclopentane bivalents à tétravalents, identiques ou différents,
A⁵ représente des radicaux alkyle, alkoxy or cycloalkyle saturés ou insaturés oléfiniquement, identiques ou différents, ayant chacun 1 à 16 atomes de carbone, des radicaux cholestane, des radicaux cholestéryle, des atomes d'halogène, des atomes d'hydrogène, des groupes hydroxyle, nitrile, acryloxy, (méth)acryloxy, (méth)acryloxyéthylèneoxy, (méth)acryloxydi(éthylèneoxy), (méth)acryloxytri(éthylèneoxy), R- ou S-tétrahydrofuranecarboxylate et trialkylsiloxy, dont les radicaux alkyle possèdent chacun 1 à 8 atomes de carbone,
R représente des radicaux hydrocarbure en C₁-C₁₈ éventuellement substitués, identiques ou différents,
a, b, c, d, f, g, h, i et k représentent chacun des nombres entiers, identiques ou différents, ayant une valeur de 0 à 3, la somme a+b+c+d+e+f+g+h+i+k s'élevant au moins à 2 et la somme de d et i au maximum à 4,
e représente un nombre ayant une valeur de 0 ou 1,
o représente un entier ayant une valeur de 0 à 3,
p représente un entier ayant une valeur de 0 à 3 et une valeur moyenne de 0,8 à 2,2 et
q représente un entier ayant une valeur de 0 à 3, et la somme de o, p et q s'élève au maximum à 3.

2. Procédés de préparation d'organosiloxanes cristallins liquides selon la revendication 1, dans lesquels, dans les radicaux de formule générale 2, n représente un entier ayant une valeur de 2 à 20, caractérisés en ce que l'on fait réagir des organosiloxanes, composés de motifs de formule générale 14,
[RₚH_{q+o}SiO_{(4-o-p-q)/2}] (14),
et/ou des organosilanes de formule générale 15
RᵣHₛ₊ₒSiY_{(4-o-r-s)/2} (15),
avec des composés de formule générale 16, 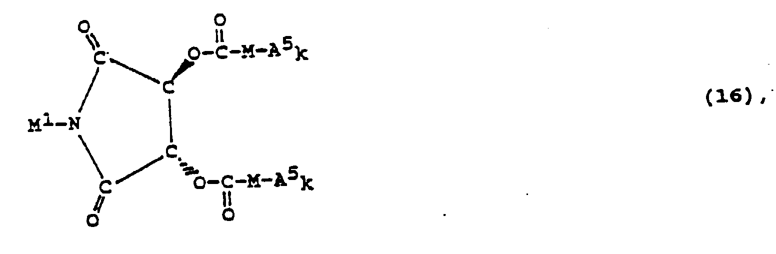 et éventuellement des composés de formule générale 17,
R²-(X¹ ₐ-A¹ _{b}-A² _{c})_{d}-Zₑ-(-X² _{f}-A³ _{g}-A⁴ ₕ-)ᵢ A⁵ ₖ (17),
et, si des organosilanes de formule générale 15 sont utilisés, les organosilanes obtenus de formule générale 18
BₒRᵣHₛSiY_{(4-o-r-s)/2} (18),
sont condensés,
où, dans les formules 14 à 18 ci-dessus
M¹ représente un radical de formule générale 19,
R²-(X¹ ₐ-A¹ _{b}-A² _{c})_{d}-Zₑ-(-X² _{f}-A³ _{g}-A⁴ ₕ-)ᵢ (19),
Y représente un groupe condensable,
R² représente un radical de formule CₙH^{m-1}, dans laquelle
m¹ a la valeur 2n-1 ou 2n-3, et
r et s représentent chacun un entier ayant une valeur de 0 à 3, la somme de o, r et s s'élève au maximum à 3 et o, p, q, M, X¹, X², A¹, A², A³, A⁴, A⁵, a, b, c, d, e, f, g, h, i, k, Z, B et R ont les significations indiquées à la revendication 1.

3. Mélanges des organosiloxanes cristallins liquides selon la revendication 1 les uns avec les autres et avec d'autres matériaux cristallins liquides.

4. Utilisation des organosiloxanes cristallins liquides selon la revendication 1 ou des mélanges selon la revendication 3 en tant que matériaux chiraux pour filtres, à des fins décoratives et dans des éléments optiques.

5. Composés de formule générale 16, 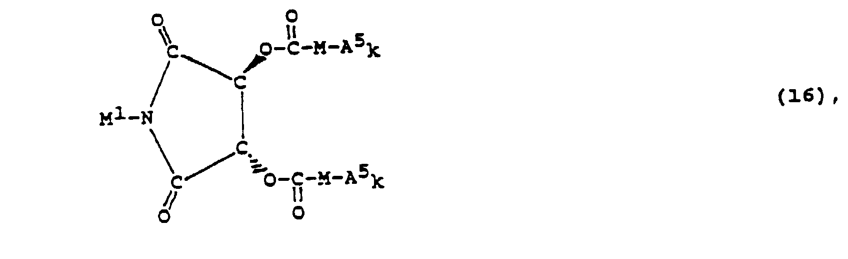 dans laquelle M, M¹, A⁵ et k ont les significations indiquées aux revendications 1 et 2.

6. Organosilanes de formule générale 18,
BₒRᵣHₛSiY_{(4-o-r-s)/2} (18),
dans lesquels o représente un entier ayant une valeur de 1, 2 ou 3 et B, R, Y, r et s ont les significations indiquées aux revendications 1 et 2.
